# EUROPEAN PATENT APPLICATION

(11) **EP 3 817 077 A1**
(43) Date of publication of application: **05.05.2021**
(21) Application number: 19838452.1
(22) Date of filing: 13.07.2019
(51) Int. Cl.: H01L 41/333, C04B 35/491, C04B 38/06, H01L 41/113, H01L 41/187, H04R 17/00

(54) **POROUS PIEZOELECTRIC MATERIAL MOLDED BODY, METHOD OF MANUFACTURING SAME, AND PROBE USING SAID MOLDED BODY**

(30) Priority: 17.07.2018 JP 2018134154; 17.07.2018 JP 2018134157; 12.11.2018 JP 2018211945; 23.05.2019 JP 2019096657; 12.07.2019 JP 2019129803
(71) Applicant: Nagai, Kiyoshi, Saitama-shi, Saitama 331-0064 (JP)
(72) Inventor: Nagai, Kiyoshi, Saitama-shi, Saitama 331-0064 (JP)
(74) Representative: Regimbeau
(86) International application number: PCT/JP2019/027827
(87) International publication number: WO 2020/017478

(57) **Abstract**

[Object] To provide a porous piezoelectric material molded body highly useful as a constituent material of a piezoelectric transducer suitable, in particular, for a probe of medical ultrasound diagnosis equipment. [Solution] A porous piezoelectric material molded body, in which 1000 or more spherical pores with an average pore diameter in the range of 2 to 70 µm are dispersedly formed per volume of 1 mm3, is characterized in that there is substantially no pore with a pore diameter larger than 50 µm, and 80 % by volume or more of the total pores that constitute a spherical pore group have a pore diameter within ± 20 % of the average pore diameter.

## Description

### [Technical Field]

The present invention relates to a porous piezoelectric material molded body useful as a constituent material of a piezoelectric transducer, and a method of manufacturing the same. More particularly, the present invention relates to a molded body of porous piezoelectric material highly useful for a piezoelectric transducer that is a main component of an array probe used in medical ultrasound diagnosis equipment, a method of manufacturing the same, and a probe using the molded body.

### [Background Art]

There are many known inorganic and organic piezoelectric materials capable of converting mechanical oscillations (amplitudes) to electrical signals or electrical signals to mechanical oscillations (amplitudes), or capable of converting electrical signals to mechanical oscillations (amplitudes) and then converting reflected waves thereof back to electrical signals. Representative applications of such piezoelectric materials can be found in (a) oscillators and (b) transducers (the terms are not clearly distinguished and are almost synonyms).
(a) As a familiar example that uses an oscillator, there may be cited electronic quartz clocks (including watches and other timepieces) with a built-in crystal oscillator that uses an artificial single crystal quartz (quartz) piezoelectric material. The quartz clock uses the oscillation of a quartz crystal to keep time, and therefore is far more accurate than conventional mechanical clocks that use a mainspring and gears.
   This oscillator is characterized in particular by being encased in an airtight enclosure so that the vacuum environment eliminates adverse effects of gas molecules on the oscillation of the crystal oscillator, thereby improving the accuracy.
   Besides, because of the advantage of being able to diagnose patients without invasion, ultrasound probes used in medical ultrasound diagnosis equipment have evolved and made an immense contribution to various diagnoses in internal medicine, surgery, ophthalmology, and the like.
   In addition to bimorph polycrystalline piezoelectric ceramics, recently, single crystal piezoelectric materials are used for the ultrasound probes. The thickness of the elements is determined by the oscillation frequency and the physical properties specific to the piezoelectric material used. High frequency ultrasound probes are designed to have a thickness of several tens of microns to 300 µm and a width of 40 to 60 % of the thickness, which necessitates ultrafine processing.
(b) As to transducers, inorganic or organic piezoelectric materials are widely used for various industrial applications in so many fields. Examples of the applications include various actuators such as level gauges, traffic volume and speed detectors, thickness meters, fish finders, sonars, flow and velocity meters, washers, humidifiers, welders, piezoelectric pumps, printers, accelerometers, and piezoelectric ignition devices.

The piezoelectric materials used in various fields are roughly classified as follows:
As single crystal piezoelectric materials, quartz, lithium niobate and the like have been known. Recently, single crystal piezoelectric materials using various so-called relaxor materials have been developed for probes used in medical ultrasound diagnosis equipment. One example of such single crystal piezoelectric materials is a single crystal (commonly known as PMN-PT single-crystal) obtained by mixing a trace amount of a relaxor material such as PMN-PT, i.e., a compound of lead-magnesium niobate (PMN) and lead titanate (PT), with PZT (lead zirconate-lead titanate) and heating the mixture at a high temperature for a long time.
As polycrystalline ceramic piezoelectric materials, valium titanate, lead titanate, and lead zirconate-lead titanate (PZT, two-component PZT) are well known. Recently, encouraged by the progress of research and development of PMN-PT single crystal and its outcome, polycrystalline PZT (collectively referred to as three-component PZT), which contains the same relaxor components as PMN-PT, has been being developed to improve and enhance the piezoelectric properties. This trend is understandable given that in particular polycrystalline PZT has long been used as piezoelectric materials for industrial and medical applications.
As organic piezoelectric materials, polyvinylidene fluoride (PVDF) is known.

What can be said about these piezoelectric materials in common is that they cannot be brought into practical use unless piezoelectric effects appropriate to their applications can be expected.

Specifically, in the medical fields, high relative permittivity (εs), high electromechanical coupling coefficient (Kt), high piezoelectric constant (d33), and the like are required.

In these days, the trend of probes for medical ultrasound diagnosis equipment is shifting to those using single crystal piezoelectric materials with excellent piezoelectric properties.
(1) Single crystallization has brought great improvements never seen before in the properties of piezoelectric materials.
   That is, very high relative permittivity (εs), high electromechanical coupling coefficient (Kt), high piezoelectric constant (d33) and the like have been confirmed.
(2) Further, the frequency bandwidth is exceptionally superior and wide compared to conventional polycrystalline PZT.
(3) The sensitivity has been improved by 4 to 8 dB in combination with the item (2).
(4) On the other hand, these single crystal piezoelectric materials are very brittle with extremely poor workability.
(5) In addition, because of the slow crystal growth, the single crystal piezoelectric material takes a long time to be commercialized, and the shape is restricted. As a result, the raw materials are very expensive. Besides, the single crystal piezoelectric materials are mechanically very brittle, resulting in poor workability. As an aside, the price of the single crystal piezoelectric materials is 5 to 8 times higher than that of polycrystalline PZT which has been commonly used for probes (as a substrate of the same shape with a plated finish).
(6) In addition to (5), in the manufacturing process, the process yield of probes is extremely poor for similar reasons.

Described below are the reasons why the trend of probes is toward the single crystal piezoelectric materials in spite of all the mentioned disadvantages.

First, the frequency bandwidth is much wider than that of conventional PZT ceramic piezoelectric materials, not to mention the significantly improved piezoelectric properties. Accordingly, while it is necessary to prepare two probes of 2.5 MHz and 5 MHz in the conventional case, only one 3.5 MHz probe can cover both frequencies in the case of the single crystal piezoelectric materials. Considering that the price of a probe is about tens of thousands to hundreds of thousands of yen, the economic advantages are outstanding. It is also a major reason that the improved sensitivity has made it possible to capture high-quality images of the deep part of the human body.

Incidentally, the frequency of the ultrasound pulses used for diagnosing a living body is determined in consideration of the depth from the body surface to an organ and the attenuation of the ultrasound waves. Generally, diagnosis is performed using a central frequency of 2 to 5 MHz in cardiovascular and abdominal regions, 5 to 7.5 MHz in children, mammary glands and peripheral sites, and 10 to 30 MHz in blood vessels.

As mentioned earlier, polycrystalline piezoelectric ceramics tend to exhibit inferior electrical properties to single crystal piezoelectric ceramics; however, they have been continuously improved in recent years. In particular, relaxer components effective for the crystallization of single crystal piezoelectric materials have had a dramatic effect on improving the properties of polycrystalline piezoelectric ceramic materials. That is, the polycrystalline piezoelectric ceramic materials of three-component PZT, obtained by adding relaxor components to conventional PZT, now have properties very close to the properties of the single crystal PMN-PT. Especially they exhibits quite interesting electrical properties.

Specifically, the polycrystalline piezoelectric ceramic materials are at almost the same level as the single crystal materials in terms of frequency bandwidth and is superior in terms of pulse characteristics.

On the other hand, the polycrystalline piezoelectric ceramics are made porous as an approach to further improvement. Such porous piezoelectric ceramics have been vigorously developed using polycrystalline piezoelectric ceramic materials as the base material, and have already been turned to practical use in some fields such as fish finder and sonar.

Since the porous piezoelectric ceramics have a relatively low frequency range of 200 to 500 KHz, when used as a transducer, it has a shape with several centimeters in diameter Φ and width. Therefore, restrictions on the formation of pores are not so strict. Besides, the average value is reflected in the characteristics, so that the frequency band is wide and the short pulsatility exceeds the characteristics of the conventional transducer. Presumably, this contributes to differentiation from the conventional model.

However, the practical application has not yet been achieved at present for array element probes used as medical probes.

Next, porous piezoelectric materials will be described.

The porous piezoelectric ceramic materials have a long history of development. Non-Patent Document 2 provides a relatively comprehensive research report, and describes a method of forming pores as follows:
(a) Using a methacrylic resin with a diameter Φ of 100 µm, 0 to 20 wt% of the resin is mixed with PZT raw material, which is pressed and then calcined to observe a change in porosity with respect to the calcination temperature. This is one approach to forming pores in piezoelectric materials.
(b) Non-Patent Document 2 mentions a method of forming pores in which the crystal size of PT (lead titanate) as a piezoelectric raw material is kept in the range of about 44 to 75 µm, PZ (lead zirconate) is prepared in four different crystal sizes, and they are mixed in a composition suitable for the target PZT. The mixture is calcined at different calcination temperatures so that pores are formed by the difference in the expansion coefficients of both crystals. After making a porous piezoelectric material in this manner, the relationship with various electrical properties (εs, d33, g33, Y33, etc.) of the porous PZT piezoelectric material obtained by the method (a) was examined in a systematic way. The experimentation was conducted in a region with a porosity of 20 % or more. Although it is described that the pores in the calcined body are 70 to 120 µm, no description is found about the dispersibility and uniformity.

In addition, as to the calcined body of porous PZT piezoelectric material obtained by the method (b), the relationship between the crystal size and various electrical properties (εs, d33, g33, S33, etc.) was examined in a systematic way in a region where the porosity was almost uniform. It is a very unique idea to use raw materials with different crystal sizes to form pores by the difference in their expansion coefficients. It is described that the pores present in the piezoelectric material are distributed in the range of 30 to 60 µm; however, there is no description about their shape and dispersion state as well as the control thereof.

Similarly, Patent Document 5 describes the electrical and mechanical properties of porous piezoelectric materials. Specifically, the content refers to the porosity and various properties (piezoelectric strain constants, tensile strength, and acoustical impedances) of pores for each of their sizes in a region where pores have a diameter of 20 to 105 µm. Presumably, it assumes a large sensor as pores have a relatively large diameter. In any case, no description is given of the state of the pores.

Patent Document 1 describes that a piezoelectric transducer (piezoelectric element) utilizing the piezoelectric effect of a piezoelectric ceramic material is used as an electromechanical conversion element in various fields, and that the piezoelectric transducer is used as an ultrasound transducer for medical ultrasound diagnosis equipment to acquire information in a living body. Patent Document 1 also describes that the medical transducer made of piezoelectric ceramics has a drawback in providing short pulses required to increase the resolution, and is not suitable for transmitting/receiving ultrasound waves in a wide frequency band. According to Patent Document 1, it is theoretically and experimentally confirmed that the use of porous piezoelectric ceramics is effective for an improvement to solve such a problem. Patent Document 1 discloses a method for producing a porous piezoelectric ceramic molded body (i.e., a porous molded body of piezoelectric ceramics) comprising the steps of mixing a piezoelectric ceramic powder with a different type of ceramic powder as a pore-forming material to obtain a ceramic powder composition, and calcining it to remove the pore-forming material.

By the method for producing a porous piezoelectric ceramic molded body described in Patent Document 1, the molded body of powdered piezoelectric ceramics is produced by calcination (i.e., sintering). Accordingly, although it is not particularly described, it can be considered that the molded body is of polycrystalline piezoelectric ceramics.

Patent Document 2 discloses a method of manufacturing a porous piezoelectric element with uniformly distributed fine pores, having a high mechanical strength and excellent workability. Specifically, Patent Document 2 describes a method for obtaining a porous piezoelectric element, in which a mixed solution of a piezoelectric material powder, a binder, and a heat-vaporizable (i.e., combustible) pore-forming material is sprayed into hot air together with compressed air to form a composite powder containing the pore-forming material the surface of which is coated with the piezoelectric material powder, and the composite powder is then calcined after molded.

Patent Document 2 also describes that a porous piezoelectric element having fine independent pores can be obtained by using the above-mentioned manufacturing method because the pore-forming materials are not combined and agglomerated. However, there is no description about the uniformity of the size of many pores formed inside the porous piezoelectric element. It is only mentioned in one embodiment that a polymethylmethacrylate resin sphere used as the pore-forming material has an average particle diameter of 5 µm.

In Patent Document 3, with reference to the paragraphs of "Means to Solve the Problem" for the problem to be solved, the content only mentions target values of a preferred form of piezoelectric materials suitable for use as a material for high-frequency piezoelectric transducers, but does not refer to any concrete solution.

Specifically, Patent Document 3 teaches a porous dielectric sheet (which may correspond to a sheet-like porous polycrystalline piezoelectric material molded body) with a thickness in the range of 0.05 to 2.0 mm, wherein the target piezoelectric material contains exclusively pores with a pore diameter of 25µm or less, the average value of the diameters of pores, each having a diameter in the range of 5 to 25 µm, is in the range of 1/1000 to 1/10 of the sheet thickness, and the apparent density is in the range of 5/10 of the true density. However, it does not teach or suggest a specific method or means to solve the most significant problem.

Besides, as a method of manufacturing a porous dielectric sheet, Patent Document 3 cites a manufacturing method disclosed in Patent Document 2, i.e., a method including the steps of spray-granulating a mixture of lead zirconate titanate powder with an average particle diameter in the range of 0.1 to 2.0 µm, a binder, and heat-decomposable particles with an average particle diameter in the range of 5 µm (preferably with a narrow distribution range of particle diameter) with a gas under pressure to produce granular powder, after molding the granular powder, calcining the molded body at a temperature equal to or higher than the thermal decomposition temperature of the heat-decomposable particles to obtain a porous calcined body, and cutting or polishing the porous calcined body into a predetermined thickness. Patent Document 3 only mentions that the heat-decomposable particles preferably have a narrow distribution range of particle diameter, and the porous dielectric sheet of the invention with uniformly distributed pores is excellent as a material for array piezoelectric transducers.

However, regarding the polymethyl methacrylate resin powder of the heat-decomposable particles used in one embodiment, there is a description that the average particle diameter is 10 µm. As to the diameters of pores of the dielectric sheet obtained in this embodiment, it is only mentioned that pores with a diameter in the range of 5 to 15 have an average diameter of 10 µm.

As already mentioned, ultrasound transducers are used in a very wide range of fields for industrial applications. In particular, in the medical field, ultrasound diagnosis has been widely adopted in both surgery and internal medicine because it can be performed non-invasively, and tomographic images of internal organs and blood vessels can be easily obtained in real time.

Furthermore, recently, two-dimensional array probes have been developed and practically used for the purpose of capturing three-dimensional volume images of an organ or an intrauterine fetus.

Non-Patent Documents 1, 7 and the like describe an array prove using arrays of a large number of strip-shaped piezoelectric transducer elements with a width of about 0.15 mm, a height of about 0.25 mm, and a length of about 10 mm, which are fixed in parallel on the surface of a sheet-shaped backing material, and covered with an acoustic matching layer and an acoustic lens. However, in non-patent document 1, there is no description about the height (or thickness) of the strip-shaped piezoelectric transducers. Generally, the piezoelectric material is used in the longitudinal vibration mode in the array probe. There is a geometrical dimensional constraint as a requirement for causing the piezoelectric material to vibrate in the longitudinal vibration mode. That is, in order to vibrate the element vertically, the frequency used is first determined. The height (or thickness) that defines the frequency of the longitudinal vibration mode is determined based on the frequency characteristics of the piezoelectric material.

Once the height is determined, the width of the element needs to be set to 0.6 times or less, more preferably 0.4 times or less the height, according to the longitudinal vibration mode design standards well known in the art. Therefore, the element is designed within this constraint. It is presumed that the dimensions of the strip-shaped piezoelectric transducer elements described above were determined in accordance with the design standards.

In order to capture a three-dimensional image, it is necessary to set the arrayed elements formed of the piezoelectric material in two dimensions (XY plane). In this case also, the design criteria of each constituent element must satisfy the above constraints.

### [Prior Art Document]

### [Patent Document]

[Patent Document 1] Japanese Unexamined Patent Application Publication No. H2-90579
[Patent Document 2] Japanese Unexamined Patent Application Publication No. H4-300253
[Patent Document 3] Japanese Unexamined Patent Application Publication No. 2000-119063
[Patent Document 4] Japanese Unexamined Patent Application Publication No. S63-78700
[Patent Document 5] Japanese Unexamined Patent Application Publication No. H1-172281

### [Non-Patent Document]

[Non-Patent Document 1] "Basics and Equipment of Ultrasound, 4th Edition", Published by VECTOR CORE Inc., January 10, 2013
[Non-Patent Document 2] "Effect of Porous Structure to Piezoelectric Properties of PZT Ceramics" K. Hikita, K. Yamada, M. Nishioka and M. Ono, R&D Laboratory, Mitsubishi Mining and Cement Co., Ltd., JJAP vol. 22 (1983), pp. 22-2 pp. 64-66
[Non-Patent Document 3] "Effect of size distribution on the relation between coordination number and void fraction of spheres in a randomly packed bed", M. Suzuki et al., Advanced Powder Technology vol. 10 (1999), pp. 353-365
[Non-Patent Document 4] "Effect of size distribution on compressive property of fine powders", M. Suzuki et al., Journal of Chemical Industry, The Society of Powder Technology (Osaka, May 1989)
[Non-Patent Document 5] "Effect of size distribution on tapping properties of fine powder", M. Suzuki et al., Powder Technology vol. 118 (2001), pp. 53-57
[Non-Patent Document 6] "Powder Technology Handbook", Hirota et al., The Society of Powder Technology, published by Nikkan Kogyo Shimbun (1998)
[Non-Patent Document 7] "A study of lead-based piezoelectric single crystals for ultrasonic medical transducers", Yasuharu Hosono, Toshiba Corporate R & D Center, 2004

### [Summary of the Invention]

### [Problems to be Solved by the Invention]

Although some explanations will be repeated, the sheet-shaped piezoelectric ceramic molded body, which is generally used as a constituent material of the piezoelectric transducer described so far, is a basic material. For the production of array probes used in medical ultrasound diagnosis equipment, sintered bodies (polycrystals) of piezoelectric ceramic powder have been used as the material. Nowadays, however, in order to meet various demands from the field of ultrasonic medical diagnosis, the use of single crystal piezoelectric materials having excellent electrical or electromechanical conversion characteristics is becoming the mainstream.

Since the single crystal piezoelectric material has problems in its economic efficiency and workability, the use of porous sheet-shaped piezoelectric ceramics having excellent economic efficiency and workability has been studied. Although the electrical or electromechanical conversion characteristics of the porous sheet-shaped piezoelectric ceramic have reached an overall satisfactory level, in the case of using it as an element of an array transducer, it is not possible to ensure uniform density (g/cubic cm) in the micro part related to the variation in pore size and the uniform dispersion of pores. As a result, the homogeneity of the relative permittivity, electromechanical coupling coefficient, various electrical properties, mechanical strength, and the like of each element cannot be maintained. Thus, there still remains a problem in the porous sheet-shaped piezoelectric ceramic that it has not yet reached a fully satisfactory level as a constituent material of the probe.

This is an essential problem related to the uniform mixing of two-component powder, and it has been considered as being very difficult to solve fundamentally.

At present, the only method for uniformly mixing at least two kinds of substances having different masses at the required volume ratio is to physically stir and mix them. In other words, there is no other way but to achieve the homogenization as much as possible by stirring and mixing over time. Neither the patent documents nor the non-patent documents mention this point, nor do they describe how to control the porosity. It seems that all of them have relied on this stirring and mixing method to manufacture porous piezoelectric materials. Therefore, in essence, it is inevitable that uniform density (g/cubic cm) cannot be ensured in the micro part related to the variation in pore size and the uniform dispersion of pores.

The inventor of the present invention has stepped away from the method of stirring and mixing, and succeeded in enabling the control of uniform pore diameter, uniform porosity, distance between pores, and the like by determining the porosity in the vicinity centered on the single pore-forming material, and stacking the processed products of the pore-forming material in layers in a mold for a desired product.

### [Means for Solving the Problems]

As already discussed in paragraphs [0007] to [0011], the inventor of the present invention has thoroughly studied the contents and results described in the patent documents and the non-patent documents, and thus reached the following conclusion. That is, the inventor of the present invention has concluded that the reason why a known porous sheet-shaped piezoelectric ceramic molded body, when used as a transducer element for an array probe, cannot exhibit sufficiently satisfactory electrical or electromechanical conversion characteristics and mechanical properties is as follows:
(1) Non-uniformity of the size of pores that constitute the porous structure of the porous sheet-shaped piezoelectric ceramic molded body
(2) Non-uniformity of dispersion of the pores (uncontrolled position where vacancies exist)
(3) Uncontrolled uniform distribution of porosity

As a result of examining these prior art techniques, the inventor of the present invention has found that when the pores contained in the porous piezoelectric material molded body are adjusted so that most of them have substantially the same pore diameter and the pores are in a highly uniform dispersed state (scattered state) and the porosity is artificially controlled to manufacture a porous piezoelectric material molded body, it is possible to obtain a porous piezoelectric material molded body with clearly superior electrical or electromechanical conversion characteristics and mechanical properties as compared not only to conventional dense molded bodies including piezoelectric material molded bodies and single crystal piezoelectric materials, but also to known porous piezoelectric material molded bodies. Thus, the inventor of the present invention has achieved the present invention.

In the case where powder is used in the manufacturing process, there is always a problem of powder transportation. Specifically, for example, for filling a powder of prescribed medicine in a tablet mold to produce tablets, the powder is usually made into granules using a spray dryer since the powder does not flow freely as it is, i.e., in the state of medicine powder as the raw material. It is no exaggeration to say that the fluidity of the granules allows the powder to be handled freely during the process.

In the process of manufacturing a sheet of piezoelectric material, a powder press is also used in a method called the dry method. Therefore, when this method is used, a powder of piezoelectric material as the raw material is prepared into granules in advance. In the present invention, apart from the viewpoint of powder handling, especially powder transportation, the study has been focused on the regularity of the arrangement of the granulated particles of the piezoelectric material poured into a mold for powder molding from the viewpoint of crystal engineering.

Specifically, the inventor of the present invention considered the case of achieving the maximum packing density of spheres in filling a space with spheres was considered. In general, the atoms that form a substance are spherical and are arrayed to be close-packed to form the crystal nuclei. Based on this fact, the inventor of the present invention considered the space packing ratio Φ (the ratio of the volume of spheres to the volume of a container) when the container is packed with spheres of the same diameter. It is known that, ideally, when packed spheres of the same diameter have a hexagonal close-packed structure or face-centered lattice structure in terms of crystal structure, the space packing ratio is maximum and the packing ratio Φ is about 0.74. The void fraction (the ratio of the volume of the space between spheres to the volume of the container) can be represented as follows: η = 1 - Φ, where η is the void fraction. Thus, the void fraction is about 0.26.

However, it is difficult to realize this ideal crystal structure model considering the case where a certain spatial region is closely packed with spheres in the manufacturing process in the industrial world.

Graph 1 shows a model in which particles of the same diameter selected are poured into a molding die. As shown in Graph 1, the space packing ratio and the void fraction of the particles having the same diameter calculated are 0.523 and 0.477, respectively. What is important here is that the space packing ratio is uniquely determined regardless of the shape (size) of the spheres.

Note that, in practice, considering that, n spheres of those to be close-packed are lined up on one side of a certain plane, (n-1) spheres are lined up next, then next n spheres ..., and this is repeated, and that the space has a square or cubic structure, in reality, a theoretical arrangement cannot be made on the wall surface, and there becomes a space. Accordingly, the actual space is larger than that of the above model with the close-packed void fraction. However, in any case, it can be seen that the void fraction is quite high.

Graph 2 considers and examines the size of small spheres inscribed in each particle in the space between the particles. It can be seen that a small sphere with a radius of a little over 15 % of the radius of the particle can exist in this space. This indicates that this relational expression holds regardless of the size of the particles. This table shows very important numerical values that form the basis of the present invention together with the issue of closest packing of spheres.

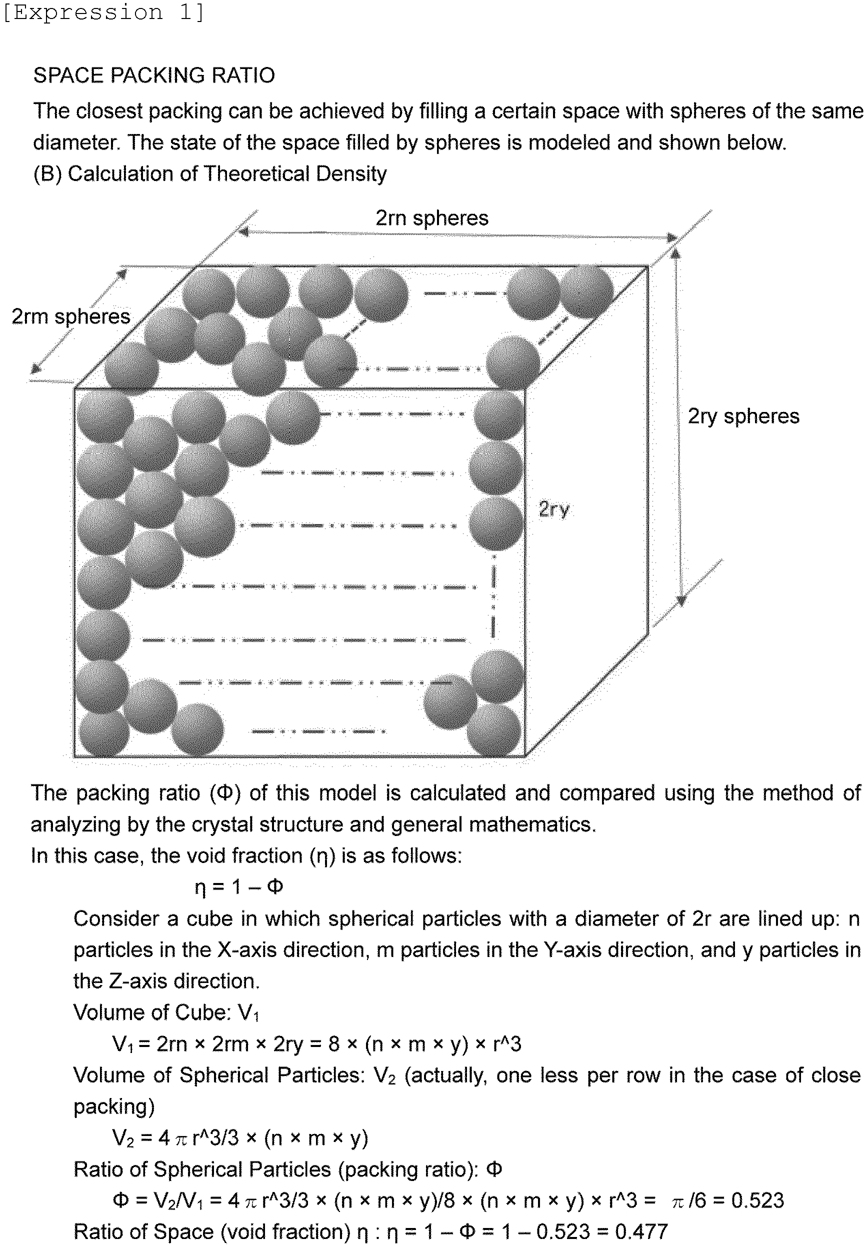

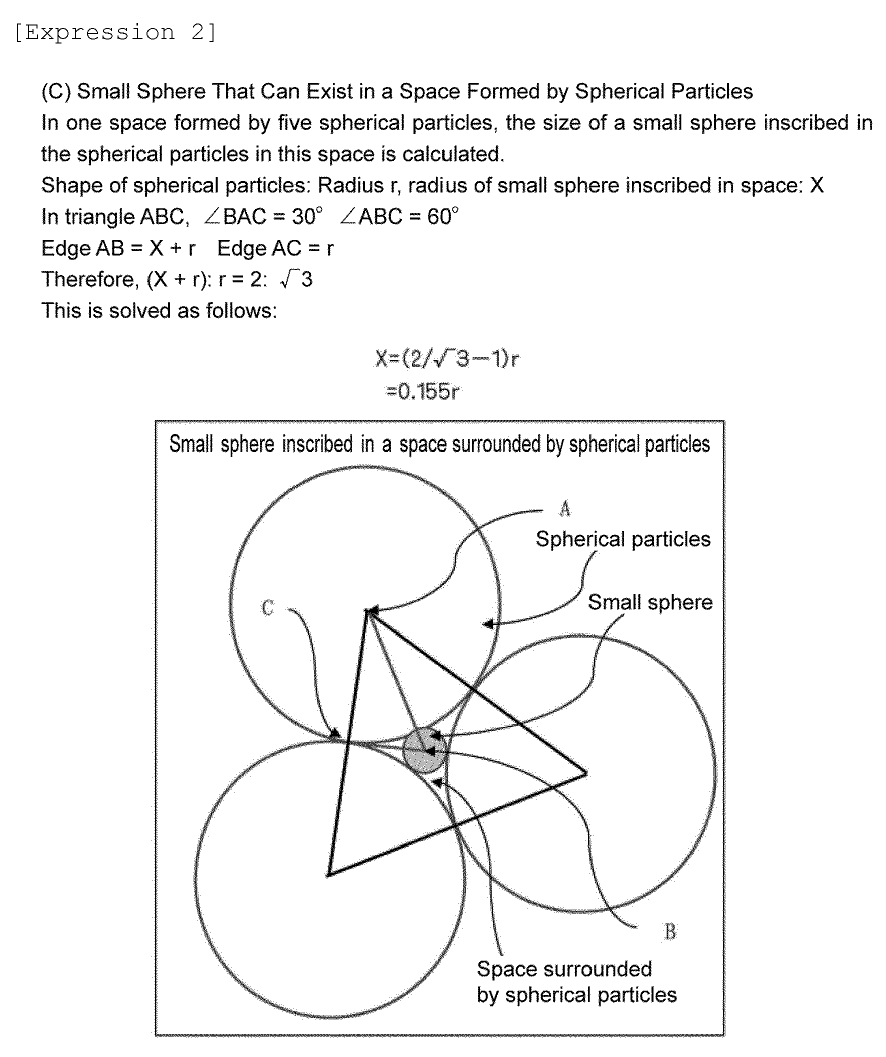

As mentioned above, theoretically or computationally, the closest packing ratio Φ of the space is 0.74 to 0.52, and the void fraction η is about 0.26 to 0.48.

The present invention focuses and is based on the following characteristics in the case where a container with a certain volume is packed with spheres of the same diameter as close as possible:
(1) the void fraction is relatively large and about 48 %, (2) voids exist very regularly, (3) the radius of a small sphere inscribed in the space created by the filling spheres is relatively small and about 15 % of the radius of the filling spheres, (4) the void fraction is relatively large, which greatly contributes to the control of the porosity of the target porous piezoelectric material within this value, (5) the spheres are arranged very regularly, (6) the initial state of being filled with the spheres is maintained in the entire subsequent manufacturing process, and the like.

Going back to the basics of crystal structure and powder engineering and understanding the characteristics, the inventor of the present invention has achieved a breakthrough invention fully capable of (a) control of the size of pores, (b) artificial control of the independence of pore position and arrangement, and (C) artificial control of the porosity.

Specifically, the inventor of the present invention has invented an epoch-making manufacturing method that enables the manufacture of a porous piezoelectric material at low cost as well as ensuring the regularity of pores present in the piezoelectric material, the uniformity of the distribution, the degree of freedom in size selection and the uniformity, and the like. In addition, the ability to design the functions of porous piezoelectric materials has increased the use of piezoelectric materials and contributed to the improvement of the functions of ultrasound sensors.

There are roughly two approaches to the process of manufacturing porous piezoelectric material products as follows:
(a) A method in which a pore-forming material is mixed in particles made of a piezoelectric material or the like. More specifically, a method that produces and uses coated composite particles consisting of a spherical pore-forming material the surface of which is coated with a fine powder of a piezoelectric material.
(b) A method in which a pore-forming material is placed in a space filled with particles made of a piezoelectric material or the like.

Both of the methods (a) and (b) are greatly different from the conventional manufacturing methods in that, having found that the geometrical composition of the pore-forming material and the piezoelectric material is secured during the entire manufacturing process from the initial setting state until the materials become a product, this characteristic is used

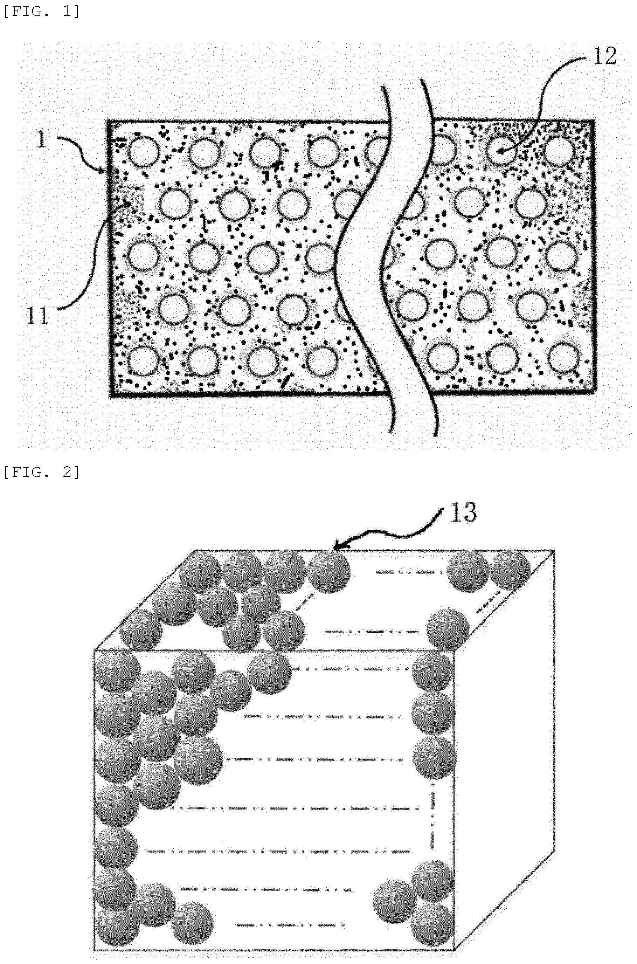

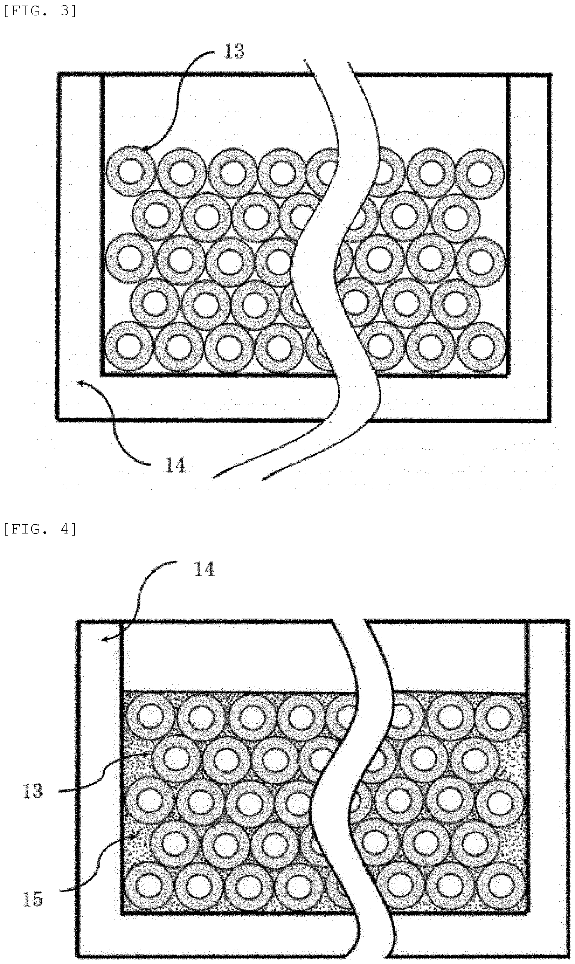

One of the proposals according to the present invention is to control pores and the porosity by using composite particles in which carbon particles of the pore-forming material illustrated in FIG. 2 are coated with a fine powder of a piezoelectric material PZT. This is a method for producing a porous piezoelectric material. Mentioned below is a void fraction P1 when a product molding die 14 illustrated in FIG. 3 is densely packed with coated composite particles 13 in which the pore-forming material is coated with a piezoelectric material, and a space or voids formed by the composite particles is filled with nothing. As is obvious from the fact that:
(a) the closest packing ratio of spheres with the same particle diameter as possible is relatively low, but the spheres are arranged regularly, and
(b) the space in which the spheres can be arranged regularly is large and the spheres are arranged geometrically, a porous piezoelectric material, in which target pores are uniformly distributed, can be manufactured.

The porosity is determined by a function of the volume ratio of the composite sphere coated with a piezoelectric material (radius: R, coating layer thickness: t) and the particles (radius: r) of the pore-forming material. That is, the ratio of the radius of the piezoelectric material and the pore-forming material artificially determines the porosity. Therefore, it has become possible to set the porosity under limited conditions. In this case, the space formed by the regularly filled coated composite particles 13 is kept hollow and disappears due to the pressure applied in the powder forming process. The porosity P₁ obtained by this method is determined by the volume ratio of the piezoelectric material PZT coated with the pore-forming material and the pore-forming material as mentioned above. The necessary mathematical expression for theoretical consideration is shown as Expression 3, and the calculation result of the porosity P₁ is shown in Table 1.

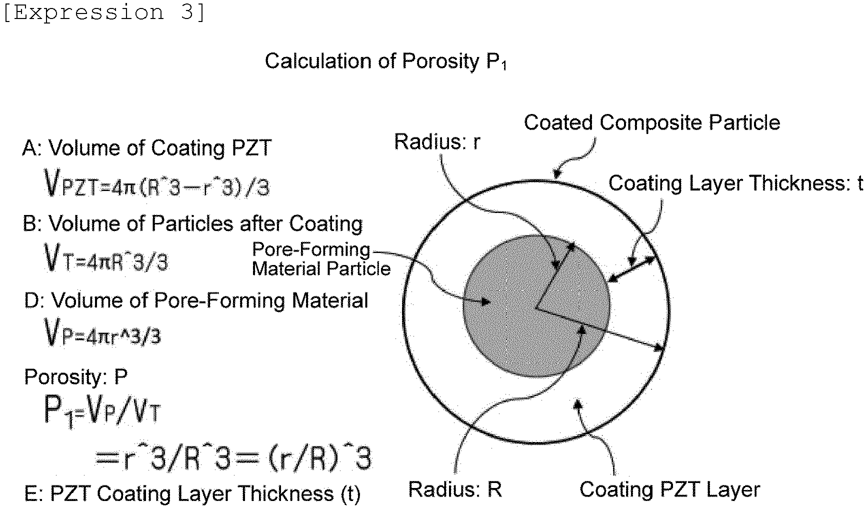

In the method described in the above paragraph, the surface of the spherical pore-forming material must be thickly coated with a piezoelectric material in order to obtain a relatively low porosity. However, it can be difficult to make the coating thicker than a certain thickness, and in some cases, problems such as peeling of the piezoelectric coating layer occur.

According to the present invention, in order to solve the problems, the product molding die is densely filled with the coated composite particles 13 coated with a piezoelectric material, and then the space formed by the coated composite particles is filled with fine particles 15 composed of a piezoelectric material and having a maximum diameter of about 15 % of the coated composite particles.

When the ultrafine particles 15 composed only of the piezoelectric material as the raw material and the particles 13 in which the carbon particles of the pore-forming material are coated with the piezoelectric material are simultaneously poured into the workpiece molding die, the space formed between the coated composite particles is filled with the ultrafine particles 15 composed of the piezoelectric material. This solves the problems. As a result, the total amount of the piezoelectric material is larger than the amount corresponding to the void fraction. The total amount can be freely controlled by the thickness of the coated piezoelectric material. This eventually leads to the control of the porosity.

It can be seen that applying this process to the manufacture of porous piezoelectric materials can eliminate all of the drawbacks and defects in conventional manufacturing, and this is a very epoch-making manufacturing method.

That is, in the conventional manufacturing process, the size and distribution of pores could not be controlled at all, which was a big bottleneck for commercialization despite of the good properties. By introducing the method of the present invention, under the conditions where the porosity is controlled, the size, uniformity and distribution of pores, and the like can be freely functionally designed according to the purpose.

The theoretical calculation method of the porosity in this case is shown in (Expression 4), and the calculation result of the porosity P₂ is also shown in (Table 1).

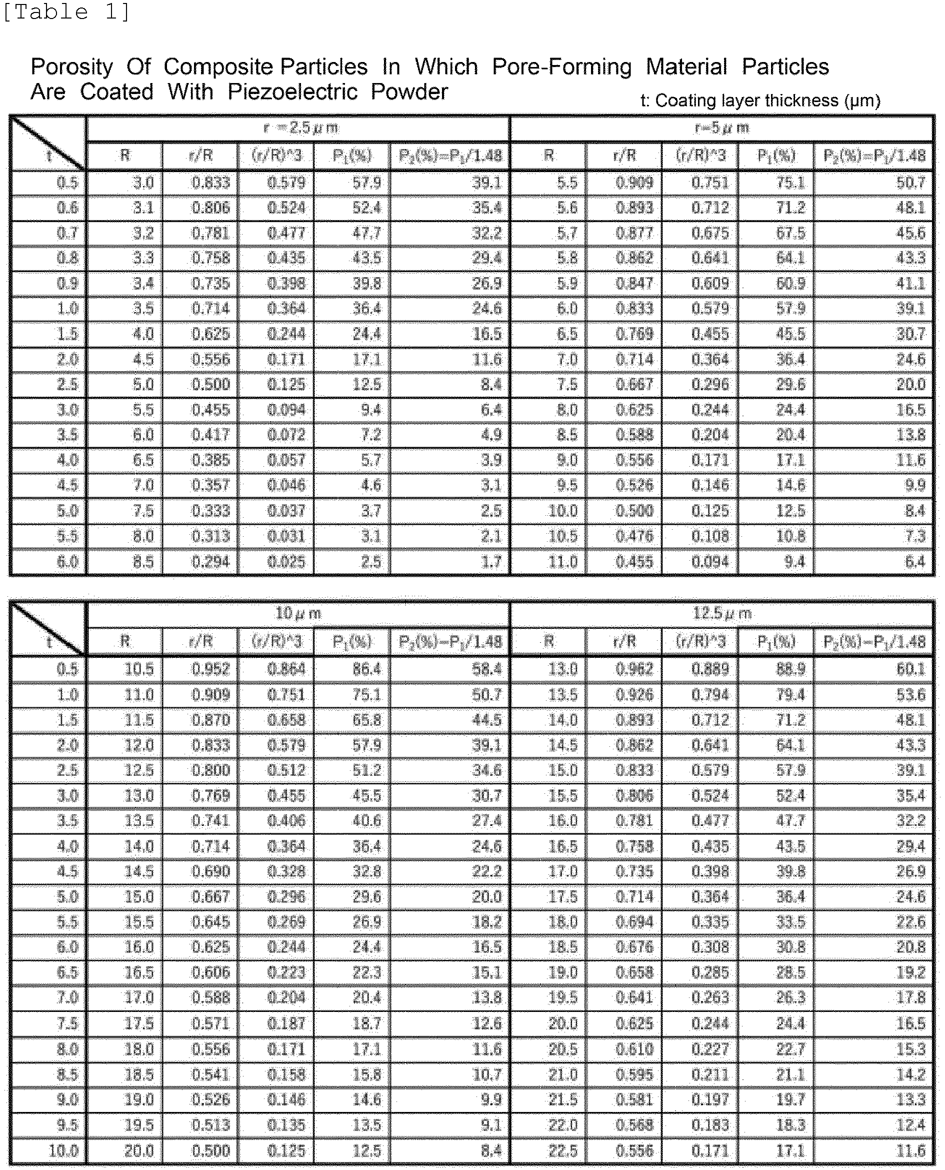

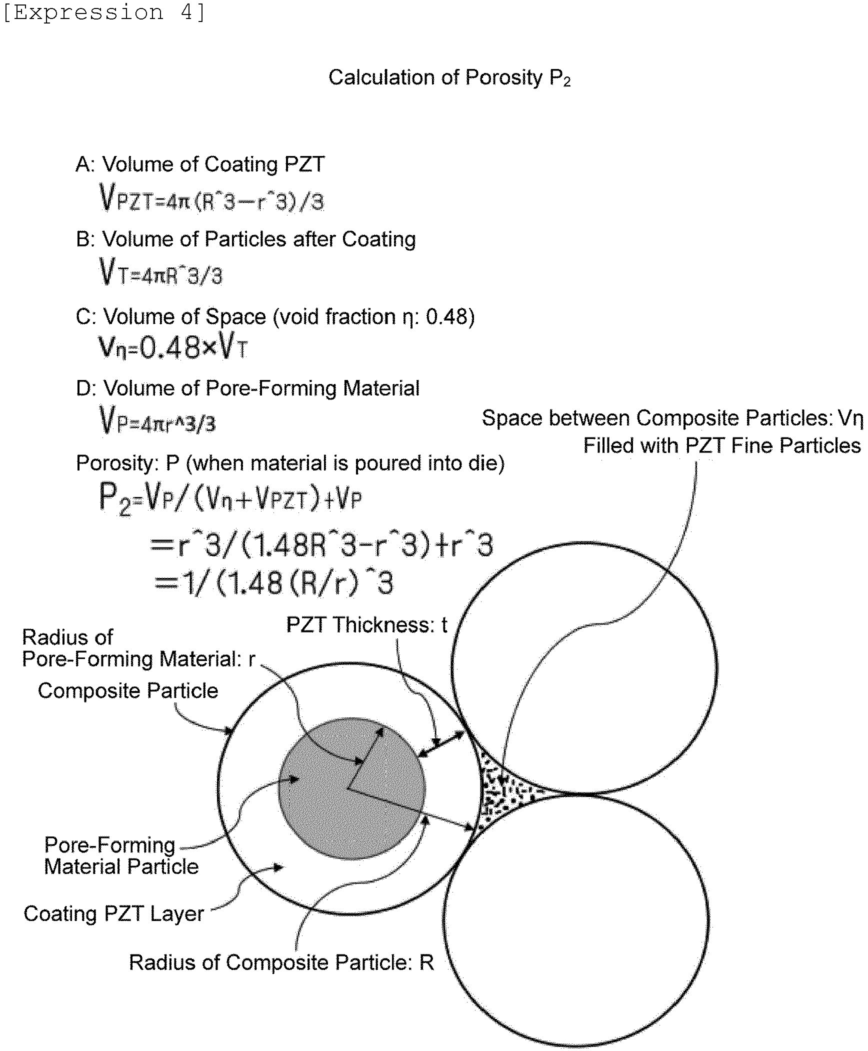

According to the present invention, a porous piezoelectric material molded body, in which 1000 or more spherical pores with an average pore diameter in the range of 2 to 50 µm are dispersedly formed in the piezoelectric material per volume of 1 mm3, can be produced in the manner described above. In the porous piezoelectric material molded body, the number of pores with a pore diameter larger than 50 µm is 1 % or less (preferably 0.5 % or less) on a number basis, and 80 % by volume or more of the total pores that constitute a spherical pore group have a pore diameter within ± 20 % of the average pore diameter.

Incidentally, in the porous piezoelectric material molded body of the present invention, the average pore diameter of spherical pores, the number of spherical pores per 1 mm3 of volume, the percentage of the number of pores having a pore diameter larger than 50 µm, the total volume of the pores constituting a spherical pore group, and the pore diameter of each spherical pore can be measured by X-ray CT observation of the porous piezoelectric molded body. In other words, each of the above measured values can be obtained by image analysis of a three-dimensional image of the porous piezoelectric material molded body acquired by X-ray CT.

The above can also be checked by cutting the porous piezoelectric material molded body and polishing the cut surface, and then examining the distribution of the pore diameters of the pores appearing on the cut surface using a scanning electron microscope (SEM).

Note that, in the present invention, the term "spherical pore" does not refer to a spherical pore in a mathematical sense, but means a pore having a shape called spherical in general.

In the porous piezoelectric material molded body of the present invention, it is desirable that "the distance between two spherical pores adjacent to each other via a piezoelectric material region be substantially the same over the entire region". It is also desirable that pore groups present inside the porous body exist independently without being offset inside the porous body in each of the vertical, horizontal, and front-rear directions.

In the above sentence "the distance between two spherical pores adjacent to each other via a piezoelectric material region be substantially the same over the entire region", the term "substantially the same distance" refers to that the distance between adjacent spherical pores falls within the range of ± 60 %, preferably within ± 40 %, and more preferably within ± 20 %, of the average distance between all adjacent pairs of the spherical pores.

The above-mentioned porous piezoelectric material molded body of the present invention can be manufactured by a method including the steps of: preparing a coated composite particle group formed of coated composite particles obtained by coating pore-forming material particles having an average particle diameter in the range of 2 to 70 µm, each having the average particle diameter, with a mixture of a piezoelectric material powder having an average diameter in the range of 1/100 to 1/5 of the average particle diameter of the particles and a binder, wherein coated composite particles with particle diameter distribution within ± 50 % of the average particle diameter of the coated composite particle group account for over 60 % by volume (preferably over 80 % by volume) of the total coated composite particles constituting the coated composite particle group; obtaining a molded body by the pressure molding of the coated composite particle group; and calcining the molded body to remove the pore-forming material particles and the binder and sintering it.

In particular, the porous piezoelectric material molded body of the present invention can be manufactured reliably with high efficiency by a method including the steps of: producing coated composite particles by coating pore-forming material particles having an average particle diameter in the range of 2 to 70 µm, each having a particle diameter within ± 20 % of the average particle diameter, with a mixture of a piezoelectric material powder having an average diameter in the range of 1/100 to 1/5 of the average particle diameter of the particles and a binder; subjecting the coated composite particle group to a particle diameter sorting process to collect a coated composite particle group in which coated composite particles with particle diameter distribution within ± 50 % of the average particle diameter of the coated composite particle group account for over 60 % by volume (preferably over 80 % by volume) of the total coated composite particles of the coated composite particle group; obtaining a molded body by the pressure molding of the collected coated composite particle group; and calcining the molded body to remove the pore-forming material particles and the binder and sintering it.

In more particularly, the porous piezoelectric material molded body of the present invention can be manufactured reliably with high efficiency by a method including the steps of: producing a coated composite particle group by coating pore-forming material particles having an average particle diameter in the range of 2 to 70 µm, each having a particle diameter within ± 20 % of the average particle diameter, with a mixture of a piezoelectric material powder having an average diameter in the range of 1/100 to 1/5 of the average particle diameter of the particles and a binder; subjecting the coated composite particle group to a particle diameter sorting process to collect a coated composite particle group in which coated composite particles with particle diameter distribution within ± 10 % (preferably within ± 5 %) of the average particle diameter of the coated composite particle group account for over 80 % by volume (preferably over 90 % by volume) of the total coated composite particles of the coated composite particle group; obtaining a molded body by the pressure molding of the collected coated composite particle group; and calcining the molded body to remove the pore-forming material particles and the binder and sintering it.

The present invention can be applied to a piezoelectric transducer array including an array of piezoelectric transducers made of the porous piezoelectric material molded body of the present invention, acoustic matching layer arranged on the surface of the piezoelectric transducer array, a backing material attached to the back surface of the piezoelectric transducer array, and an array transducer equipped with an acoustic lens attached to the surface of the acoustic matching layer.

In the following, preferred embodiments of the porous piezoelectric material molded body of the present invention will be described.
(1) Ninety percent by volume or more of the total spherical pores constituting a spherical pore group have a pore diameter within ± 20 % of the average pore diameter.
(2) Eighty percent by volume or more of the total spherical pores constituting a spherical pore group have a pore diameter within ± 10 % of the average pore diameter.
(3) Ninety percent by volume or more of the total spherical pores constituting a spherical pore group have a pore diameter within ± 10 % of the average pore diameter.
(4) The distance between two spherical pores adjacent to each other via the piezoelectric material is substantially the same in 80 % by volume or more of the total internal region.
(5) The average pore diameter of the spherical pore group is substantially 15 µm or less.
(6) The porous molded body is in a shape of a strip, having a height and width in the range of 0.05 to 2 mm and a length in the range of 5 to 50 mm.
(7) The piezoelectric material is made of lead zirconate titanate (two-component PZT) or three-component PZT.
(8) The apparent density is in the range of 5/10 to 9/10 of the true density, more preferably in the range of 50/100 to 99/100.
(9) The porous piezoelectric material molded body is in a shape of a sheet or a strip, and the average value of the pore diameter is within the range of 1/1000 to 1/10 of the sheet thickness or the strip thickness.

In implementing the method of manufacturing a porous piezoelectric material molded body of the present invention, in a step of obtaining a molded body by the pressure molding of a collected coated composite particle group, the molded body may also be obtained by preparing in advance fine particles with 15 % or less of the composite particle diameter using a fine powder of piezoelectric material (by making a slurry consisting of a piezoelectric material fine powder and a binder into fine granules by a spray dryer or the like), preparing a mixture of a coated composite particle group and a group of the fine particles of the piezoelectric material in a volume ratio of the former to the latter within the range of 1/1 to 1/10, and then pressure molding this mixture into a molded body. By using such a molded body manufacturing method, it has become possible to artificially adjust the porosity of the porous piezoelectric material molded body.

### [Effects of the Invention]

A porous piezoelectric material of 5 mm□ × 8 mmH obtained by the present invention was scanned with X-ray CT to observe the state of the pores. As a result of the observation, the volume of the pores was 17.4 mm3, the volume of PZT as the base material was 200 mm3, and the volume fraction of the pores was about 9 %.

A porous piezoelectric material having 2,500 pores/mm3 or more, in which pores with an average diameter Φ of 8 µm accounted for 98 %, was obtained.

As described above, in the porous piezoelectric material molded body of the present invention, a number of pores with a high uniformity of pore diameter are uniformly scattered inside the molded body made of a piezoelectric material. Therefore, the porous piezoelectric material molded body is clearly different from the conventional ones.

Table 2 is a graph of values obtained experimentally to show how each of the physical properties of the porous piezoelectric material varies according to a change in the porosity (each characteristic value for a porosity of 0 % indicates the value of a polycrystalline piezoelectric material calcined body used).

The output (sensitivity) Sp of a piezoelectric element as a sensor is determined by the voltage V applied to the sensor, the relative permittivity ε33, and the electromechanical coupling coefficient K33.

When the voltage is constant, the important factors are narrowed down to the relative permittivity ε33 and the electromechanical coupling coefficient k33.

As can be seen from the graph, the relative permittivity decreases significantly as the porosity increases, while the electromechanical coupling coefficient increases when the porosity is below a certain level.

In other words, as a sensor, the output indicates values higher than that of the base piezoelectric material when the porosity is below about 15 %. Compared to the single crystal piezoelectric material, the value is about 90 %.

On the other hand, for the design of ultrasound probes, PiezoCAD (ultrasound probe design and development support software) of Eastek Corporation offers a very high correlation between simulation values and measured values, and therefore is widely used and highly appreciated in the industry.

Using this software, for a PZT piezoelectric material as a base, porous PZT using the material with a porosity of 15 %, and a single crystal piezoelectric material, the electrical properties (frequency band characteristics and pulse characteristics) were compared in 3.5 MHz oscillation by applying the thickness vibration (note that this experimental data is obtained by estimating the characteristics of three-component PZT under the condition that the additivity is established based on the data obtained by using another PZT material).

The results are shown in Table 3. The electrical band properties are comparable to or better than those of piezoelectric transducers manufactured using single crystal piezoelectric materials, and, in terms of pulse characteristics, the result surpasses them. In addition, there are no major obstacles or difficulties in manufacturing, and mechanical properties (robustness) and workability (easiness of processing) are also excellent.

By analogizing the properties of the array type probe based on the data obtained from the thickness vibration, the following can be expected.

It has been necessary to prepare different probes for the diagnosis of different living bodies. However, as described above, if a probe having a center frequency of 3.5 MHz is prepared using the porous piezoelectric material of the present invention, only one probe can cover frequencies of 2.5 to 5 MHz.

According to Patent Document 2, a probe having preferable properties can be obtained when the porosity is 0.1 to 0.75, preferably 0.30 to 0.65, and it is not possible to take advantage of the properties of the porous piezoelectric material when the porosity is less than 0.1. On the other hand, from the results of empirical experiments according to the present invention, it was predicted that preferable characteristics would be exhibited when the porosity was 0.01 to 0.25, preferably 0.15 or less.

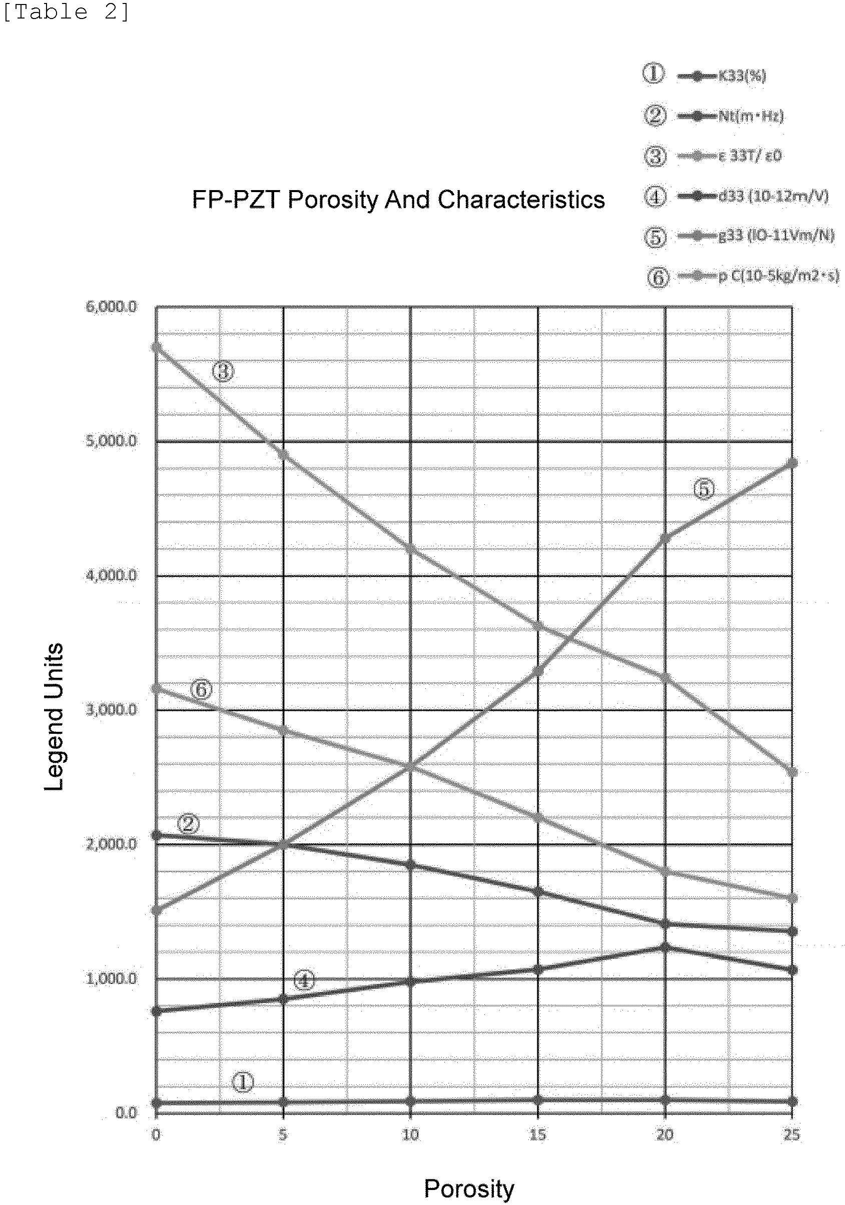

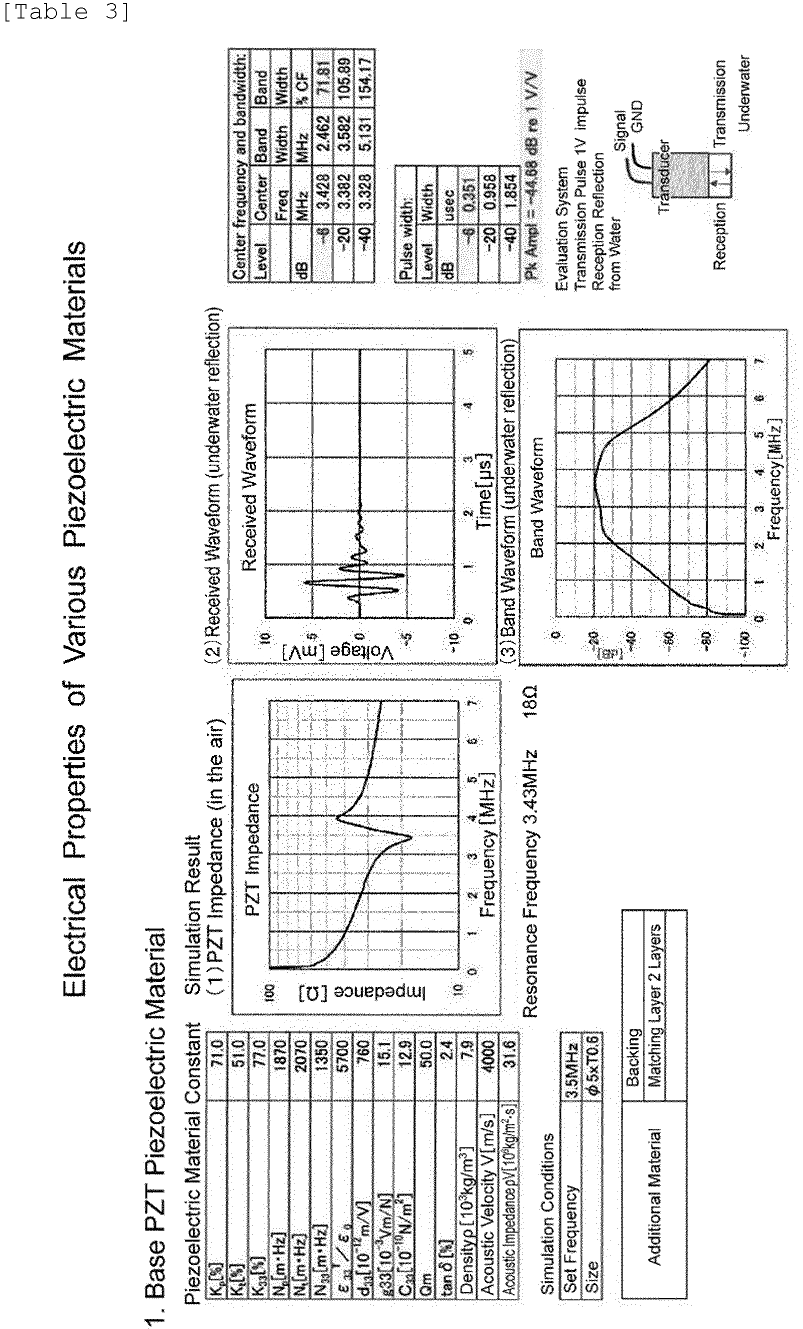

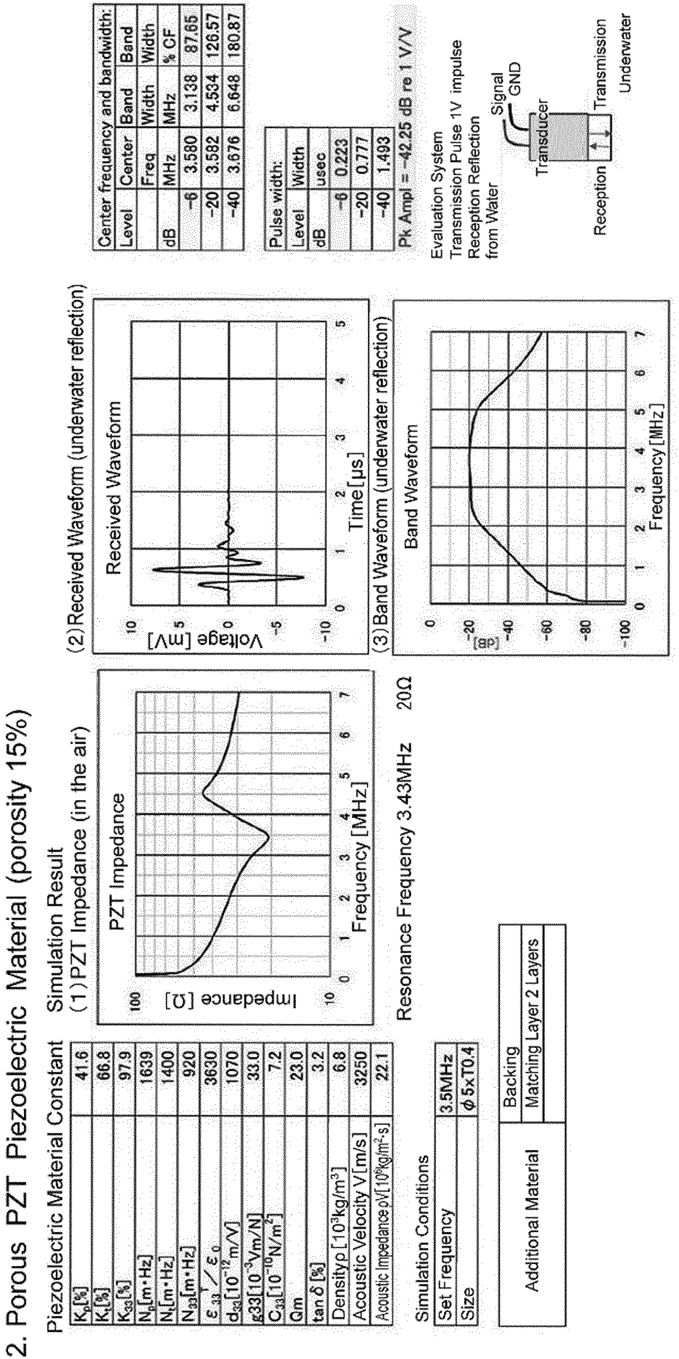

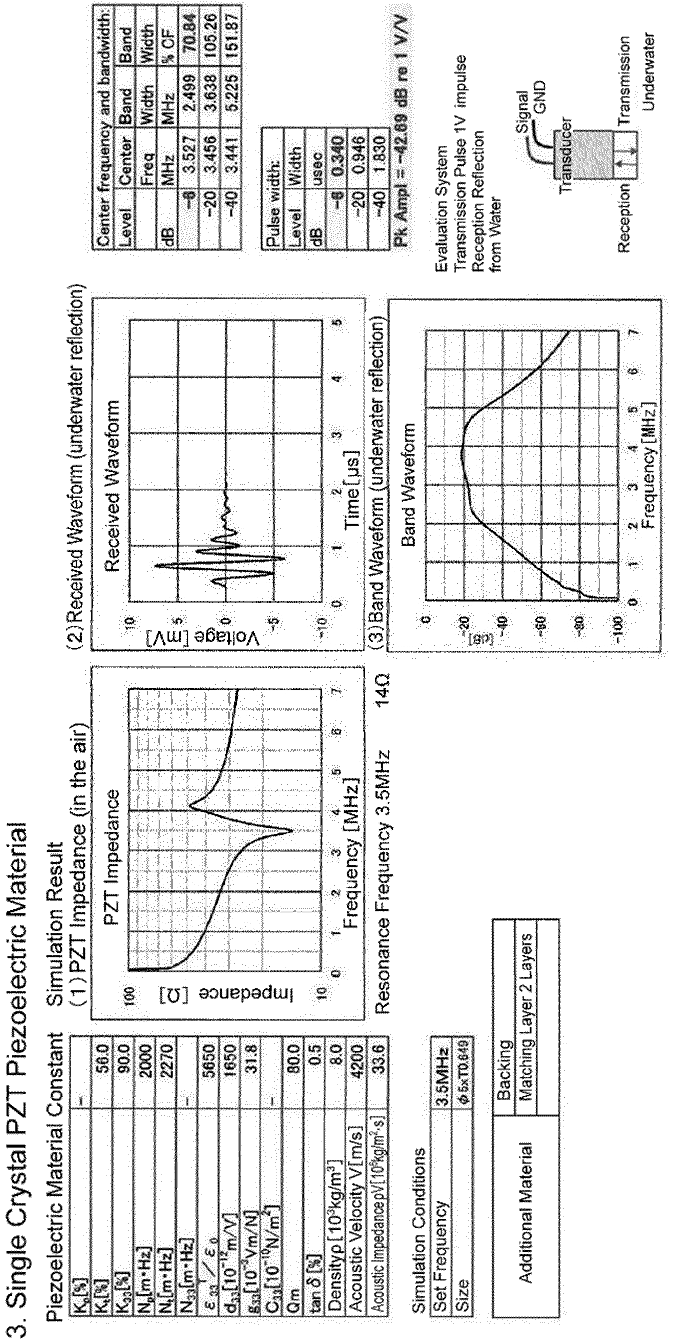

### [Brief Description of the Drawings]

[FIG. 1] FIG. 1 is a conceptual diagram illustrating an example of the structure of a porous piezoelectric material molded body according to the present invention.
[FIG. 2] FIG. 2 is a diagram for explaining the concept of obtaining a basic molded body by close-packing coated composite particles used for manufacturing the porous piezoelectric material molded body of the present invention.
[FIG. 3] FIG. 3 is a conceptual diagram illustrating, as a two-dimensional diagram, an arrangement of coated composite particles in a process of obtaining a molded body of a coated composite particle group illustrated in FIG. 2.
[FIG. 4] FIG. 4 is a conceptual diagram illustrating a process of obtaining a molded body by pressing a coated composite particle group with added piezoelectric material particles (granule) used in an example of the production of the porous piezoelectric material molded body according to the present invention.

### [Modes for Carrying Out the Invention]

In the following, a porous piezoelectric material molded body and a manufacturing method thereof of the present invention will be described in detail with reference to the accompanying drawings. FIG. 1 is a conceptual diagram illustrating an example of the structure of a porous piezoelectric material molded body according to the present invention. In FIG. 1, a porous piezoelectric material molded body 1 of the present invention includes a sintered body of piezoelectric material powder 11 and a number of fine spherical pores 12 which are scattered inside the molded body in a substantially aligned state (i.e., present as being uniformly dispersed). Although FIG. 1 illustrates a two-dimensional conceptual view, a three-dimensional conceptual view can be illustrated similarly.

As a most common example of piezoelectric material powder for obtaining a sintered body of piezoelectric material powder constituting the porous piezoelectric material molded body of the invention, lead-zirconate titanate (PZT) powder may be cited. However, the piezoelectric material powder is not limited to the two-component PZT or the three-component PZT, and any powder material that exhibit piezoelectricity can be used without particular limitation. As to usable piezoelectric material powders, a detailed description can be found in Patent Documents 1 to 3, the contents of which are incorporated herein by reference.

In addition, an aggregate of hollow piezoelectric material particles may also be used as the piezoelectric material powder.

Preferably, the piezoelectric material powder has an average diameter in the range of 1/100 to 1/5 of the average particle diameter of pore-forming material particles (described later). In other words, piezoelectric material particles constituting the piezoelectric material powder need to have a powder diameter sufficiently smaller than that of the pore-forming material particles, and an aggregate of piezoelectric material powders having a powder diameter as uniform as possible is preferred.

FIG. 2 is a conceptual diagram for explaining a process of obtaining a molded body by pressing a coated composite particle group used for manufacturing the porous piezoelectric material molded body of the present invention.

In the process of manufacturing the porous piezoelectric material of the invention, as described above, in the first step, coated composite particle powder is prepared; the coated composite particle powder is formed of a coated composite particle group obtained by coating pore-forming material particles having an average particle diameter in the range of 2 to 70 µm, each having a particle diameter within ± 20 % of the average particle diameter (i.e., particles highly uniform in particle diameter), with a mixture of a piezoelectric material powder having an average diameter in the range of 1/100 to 1/5 and a binder such that coated composite particles with particle diameter distribution within ± 50 % of the average particle diameter of the coated composite particle group account for over 60 % by volume of the total coated composite particles constituting the coated composite particle group.

As the pore-forming material particles used in the first step, synthetic resin particles such as spherical carbon powder (spherical carbon particles), spherical polymethylmethacrylate particles, and thermosetting epoxy are generally used. Other examples of the pore-forming material particles that can be used for the manufacture of the porous piezoelectric material molded body of the invention can be found in the above-mentioned Patent Documents 1 to 3, the contents of which are incorporated herein by reference.

Incidentally, the pore-forming material particles having an average particle diameter in the range of 2 to 70 µm, each having a particle diameter within ± 20 % of the average particle diameter (i.e., particles highly uniform in particle diameter), are available from companies such as Nippon Carbon Co., Ltd., Soken Chemical & Engineering Co., Ltd., Japan Exlan Co., Ltd., IBIDEN Co., Ltd., and Gun Ei Chemical Industry Co., Ltd. the pore-forming material particles can also be easily obtained, if necessary, by classifying spherical pore-forming material particles obtained from a desired company with a commercially available precision classifier (precision sieve).

Examples of the binder used in the above-mentioned first step include, but are not limited to, a mixture of polyvinyl alcohol as an aqueous solution and a water-soluble acrylic resin. Other examples of the binder can be found in detail in the above-mentioned Patent Documents 1 to 3, the contents of which are incorporated herein by reference.

The coated composite particle group coated with a mixture of a powder of piezoelectric material particles and a binder can be produced by, for example, dispersing a powder of piezoelectric particles in a binder prepared as a dilute aqueous solution to obtain a slurry, and then spray-drying the slurry and pore-forming material particles using a spray dryer such as a spray-drying atomizer. As to the process of producing a coated composite particle group coated with a mixture of a piezoelectric material powder and a binder by using spray-drying of a slurry of a binder aqueous solution, in which piezoelectric particles are dispersed, and pore-forming material particles, detailed descriptions can be found in the above-mentioned Patent Documents 2 and 3, the contents of which are incorporated herein by reference.

In the process of producing a coated composite particle group coated with a mixture of a piezoelectric material powder and a binder to manufacture the porous piezoelectric material molded body of the invention, it is preferable to appropriately adjust the concentration of the binder aqueous solution, the mixing ratio of the binder aqueous solution and the piezoelectric material powder, spray-drying (or spray-granulation) conditions, and the like to obtain a coated composite particle group with a uniform particle diameter, in which coated composite particles with particle diameter distribution within ± 50 % of the average particle diameter of the coated composite particle group account for over 60 % by volume (preferably over 80 % by volume) of the total coated composite particles of the coated composite particle group.

However, there may be a case where it is difficult to obtain a coated composite particle group, in which coated composite particles whose particle diameter distribution is within ± 50 % of the average particle diameter of the coated composite particle group account for over 60 % by volume of the total coated composite particles of the coated composite particle group by appropriately adjusting the concentration of the binder aqueous solution, the mixing ratio of the binder aqueous solution and the piezoelectric material powder, spray-drying conditions, and the like In the process of producing the coated composite particle group coated with a mixture of the piezoelectric material powder and the binder. In such case, a step of subjecting the obtained coated composite particle group to a known particle diameter sorting process can be additionally performed to collect a coated composite particle group in which coated composite particles with particle diameter distribution within ± 50 % of the average particle diameter of the coated composite particle group account for over 60 % by volume (preferably over 80 % by volume) of the total coated composite particles of the coated composite particle group.

A precision classifier such as, for example, an ultrasound precision classifier manufactured and sold by a classifier manufacturing company such as Aisin Nano Technologies Co., Ltd. can be used to perform the particle diameter sorting process.

In a case where the mixing of particles other than coated composite particles, i.e., the mixing of granule consisting of piezoelectric material alone, is disadvantageous, the above process can also be performed using an aerosol mass spectrometer manufactured and sold by KANOMAX Japan Inc.

The coated composite particle group, in which coated composite particles with particle diameter distribution within ± 50 % of the average particle diameter of the coated composite particle group account for over 60 % by volume of the total coated composite particles of the coated composite particle group is then subjected to a step of pressing to obtain a molded body.

FIG. 3 illustrates an image of a pressure molding process of a coated composite particle group coated with a mixture of a piezoelectric material powder and a binder used for the manufacture of the porous piezoelectric material molded body of the invention. As illustrated in FIG. 3, the coated composite particles 13 are placed in the molding die 14 to be subjected to pressure molding. Regarding the pressure molding of the coated composite particles 13 coated with a mixture of piezoelectric material particles and a binder, detailed descriptions can be found in the above-mentioned Patent Documents 2 and 3, the contents of which are incorporated herein by reference.

The molded body obtained by the pressure molding of the coated composite particle powder is then calcined at a temperature higher than the thermal decomposition temperature of the pore-forming material particles. The pore-forming material particles and the binder are burned and removed by the calcination. After that, the molded body is sintered by further heating it at a high temperature. As to the process of burning and removing the pore-forming material particles and the binder through the calcination of the molded body obtained by the pressure molding of the coated composite particle group and the sintering process, detailed descriptions can be found in the above-mentioned Patent Documents 2 and 3, the contents of which are incorporated herein by reference.

Note that when it is desired to obtain a porous piezoelectric material molded body with a low porosity as the one produced by the method described above, such a molded body can be obtain by preparing a mixture of a piezoelectric material fine powder and coated composite particles in a volume ratio of the former to the latter, for example, within the range of 1/1 to 1/10, and then pressure molding this mixture in the step of pressure molding the coated composite particle group to obtain a molded body. That is, the porosity of the porous piezoelectric material molded body can be artificially finely adjusted by using such a molded body manufacturing method.

A wet process may also be used for the purpose of obtaining a porous piezoelectric material molded body with a low porosity.

In this embodiment, a water-soluble binder is used to prepare a slurry of PZT fine powder for producing PZT-coated composite particles. For this reason, the coated composite particles is very sensitive to moisture. Accordingly, a hydrophobic solution such as, for example, acetone is sprayed on the surface of the coated composite particles. After the coated composite particle group whose surface has been preprocessed in this manner is placed in a desired mold, the space formed between the coated composite particles is filled with a slurry of PZT fine powder prepared by using a water-soluble binder. With this, a porous piezoelectric material molded body with a low porosity can be obtained.

The slurry of PZT fine powder may be prepared by a mixture of a piezoelectric material fine powder and coated composite particles/a water-soluble binder and a PZT fine powder in a volume ratio of the former to the latter, for example, within the range of 1/1 to 1/10. That is, the porosity of the porous piezoelectric material molded body can be freely adjusted by using such wet-process manufacturing method of a molded body.

When space formed between the coated composite particles is filled with a slurry of PZT fine powder prepared by using a water-soluble binder, the filling process can be performed more efficiently if the entire molding die is brought into a reduced pressure environment by a vacuum pump or the like.

### Example 1

As a piezoelectric material particle powder, PZT powder (two-component PZT powder) having a particle diameter in the range of 0.1 to 1 µm was selected. Next, 50 parts by mass of this PZT powder was mixed with 50 parts by mass of a binder aqueous solution (a mixture of an aqueous solution of 1 wt% polyvinyl alcohol and an aqueous solution of 1 wt% water-soluble acrylic resin) to prepare a PZT powder slurry. Besides, a spherical carbon powder having a particle diameter of about 10 µm (average particle diameter of 10 µm with particle diameter distribution within ± 50 %) was separately prepared. By spray-drying 100 parts by mass of the above PZT powder slurry and 100 parts by mass of carbon powder having a particle diameter of about 10 µm using a spray-drying atomizer, coated composite particles (average particle diameter: 20 µm) formed of PZT powder and a binder and coated with a coating layer having a thickness of about 5 µm was obtained. Then, the particle diameter distribution of the coated composite particles was measured, and it was found that it did not correspond to a coated composite particle group, in which coated composite particles with particle diameter distribution within ± 50 % of the average particle diameter of the coated composite particle group accounted for over 60 % by volume of the total coated composite particles of the coated composite particle group. Accordingly, classification was performed using an ultrasound classifier to collect a coated composite particle group in which coated composite particles with particle diameter distribution within ± 50 % of the average particle diameter of the coated composite particle group accounted for over 60 % by volume of the total coated composite particles of the coated composite particle group. The coated composite particles collected by the classification were placed in the molding die 14 as illustrated in FIG. 2, and the pressure molding process was performed by applying a pressure of 1 to 1.5 ton/cm2 to the coated composite particles to obtain a molded body. Incidentally, the presence of voids inside the molded body obtained by the pressure molding process could not be visually observed under a high-magnification microscope. The dispersion of the coated composite particle fine powder in the molding die is illustrated as a conceptual diagram in FIG. 3. Thereafter, the molded body obtained by pressing or compacting the coated composite particle group was pre-calcined in air at 450 °C for 1 hour to sublimate and remove carbon particles, and then calcined (sintered) at 1250 °C to thereby obtain a porous PZT molded body (sintered body), in which spherical pores having an average pore diameter of about 10 µm were formed in a three-dimensional arrangement at the position where the carbon particles were present. After cutting the porous PZT molded body and polishing the cut surface, the distribution of the pore diameter of pores appearing on the cut surface was examined. As a result, it was found that the pore diameters of 90 % by volume or more of pores constituting a pore group were distributed within ± 10 % of the above-mentioned average pore diameter (about 10 µm) .

It was also found that the distance between two spherical pores present adjacent to each other via a piezoelectric material region was distributed within the range of ± 20 % over 60 % of the total internal region. Then, the density of the obtained porous PZT molded body was measured. As the density was 5.25 g/cm3, when calculated in consideration of the density of PZT used (6.70 g/cm3), the porosity was found to be 22 % by volume. In addition, the number of spherical pores with an average pore diameter in the range of 2 to 50 µm formed inside the porous PZT molded body was examined by X-ray CT observation. As a result, it was found that there were 1000 or more of them per 1 mm3 of volume.

### Example 2

Using the same method as described in Example 1, a powder of coated carbon particles (average particle diameter: 14 µm) formed of PZT particle powder and a binder and coated with a coating layer having a thickness of about 2 µm was obtained. After that, classification was performed to collect a coated composite particle group in which coated composite particles with particle diameter distribution within ± 50 % of the average particle diameter of the coated composite particle group accounted for over 80 % by volume of the total coated composite particles of the coated composite particle group.

Besides, PZT fine powder particles (granules) having a particle diameter of 1 to 2 µm (a particle diameter of about 15 % of the coated composite particles) were separately prepared using PZT powder in the range of 0.1 to 1 µm.

100 parts by volume of the coated composite particles collected by the classification were placed in a raw material supply hopper. Subsequently, 50 parts by volume of the above-mentioned granules prepared separately were put in the hopper. Vibration was applied to them by a vibrator attached to the hopper. When the granules put in later disappeared, the on-off valve located at the bottom of the hopper was opened to inject the piezoelectric raw material inside the hopper into a molding die through a supply pipe.

As in Example 1, the piezoelectric raw material placed in the molding die was molded (the dispersion of the coated composite particles 13 and the PZT particles (granules) 15 in the molding die 14 is illustrated as a conceptual diagram in FIG. 4), and pre-calcined and then calcined to obtain a porous PZT molded body. The density of the obtained porous PZT molded body was measured. As the density was 6.85 g/cm3, when calculated in consideration of the density of PZT used (7.60 g/cm3), the porosity was found to be 10 % by volume.

After cutting the porous PZT molded body and polishing the cut surface, the distribution of the pore diameter of pores appearing on the cut surface was examined. As a result, it was found that the pore diameters of 90 % by volume or more of pores constituting a pore group were distributed within ± 10 % of the above-mentioned average pore diameter (about 10 µm).

It was also found that the distance between two spherical pores present adjacent to each other via a piezoelectric material region was distributed within the range of ± 20 % over 60 % of the total internal region. In addition, the number of spherical pores with an average pore diameter in the range of 2 to 50 µm formed inside the porous PZT molded body was examined by X-ray CT observation. As a result, it was found that there were 1000 or more of them per 1 mm3 of volume.

### Explanation of Symbols

- 1: Porous piezoelectric material molded body
- 11: Piezoelectric material
- 12: Pore
- 13: Coated composite particle
- 14: Molding die
- 15: Piezoelectric material fine powder particle (granule)

## Claims

1. A porous piezoelectric material manufacturing method for a powder molded body made of piezoelectric material, comprising:
under conditions that a powder molding die is close-packed with spherical piezoelectric material particles or coated composite particles obtained by coating a spherical pore-forming material with piezoelectric material,
filling a space, which is formed between the particles regularly arranged, with spherical fine particles made of piezoelectric material in a case of the coated composite particles or spherical pore-forming material particles in a case of spherical piezoelectric particles, as required, to control porosity.

2. The porous piezoelectric material manufacturing method including claim 1, wherein material filled in the space formed between the particles have a particle diameter that is 0.155 times the diameter of the particles or less.

3. The porous piezoelectric material manufacturing method including claim 1 or 2, wherein upper and lower limits of the particle diameter of material filled in the space formed between the particles is set in a range from and below 0.155 times the diameter of the particles.

4. A porous piezoelectric material molded body including claim 1, wherein
1000 or more spherical pores with an average pore diameter in a range of 2 to 70 µm are dispersedly formed in the piezoelectric material per volume of 1 mm3,
the number of pores with a pore diameter larger than 50 µm is 1 % or less on a number basis, and
80 % by volume or more of total pores that constitute a spherical pore group have a pore diameter within ± 20 % of the average pore diameter.

5. The porous piezoelectric material molded body according to claim 4, wherein 90 % by volume or more of the total pores that constitute the spherical pore group have a pore diameter within ± 20 % of the average pore diameter.

6. The porous piezoelectric material molded body according to claim 4, wherein 80 % by volume or more of the total pores that constitute the spherical pore group have a pore diameter within ± 10 % of the average pore diameter.

7. The porous piezoelectric material molded body according to claim 4, wherein 90 % by volume or more of the total pores that constitute the spherical pore group have a pore diameter within ± 10 % of the average pore diameter.

8. A method of manufacturing a molded body of the porous piezoelectric material according to claim 1, comprising the steps of:
preparing a coated composite particle group formed of coated composite particles obtained by coating pore-forming material particles having an average particle diameter in a range of 2 to 70 µm, each having a particle diameter within ± 20 % of the average particle diameter, with a mixture of a piezoelectric material powder having an average diameter in a range of 1/100 to 1/5 of the average particle diameter of the particles and a binder, wherein coated composite particles with particle diameter distribution within ± 50 % of average particle diameter of the coated composite particle group account for over 60 % by volume of total coated composite particles that constitute the coated composite particle group;
obtaining a molded body by pressure molding of the coated composite particle group; and
calcining the molded body to remove the pore-forming material particles and the binder, and sintering the molded body.

9. A method of manufacturing a molded body of the porous piezoelectric material according to claim 1, comprising the steps of:
producing pore-forming material particles coated with a mixture of a powder and a binder (coated composite particles) by coating pore-forming material particles having an average particle diameter in a range of 2 to 70 µm, each having a particle diameter within ± 20 % of the average particle diameter, with a mixture of a piezoelectric material powder having an average diameter in a range of 1/100 to 1/5 of the average particle diameter and a binder;
subjecting the coated composite particle group to a particle diameter sorting process to collect a coated composite particle group consisting of coated composite particles with particle diameter distribution within ± 10 % of average particle diameter of the coated composite particle group;
obtaining a molded body by pressure molding of the coated composite particle group collected; and
calcining the molded body to remove the pore-forming material particles and the binder, and sintering the molded body.

10. An array probe, comprising:
a piezoelectric transducer array including an array of piezoelectric transducers made of the porous piezoelectric material molded body of claim 4;
an acoustic matching layer arranged on a surface of the piezoelectric transducer array;
a backing material arranged on a back surface of the piezoelectric transducer array; and
an acoustic lens arranged on a surface of the acoustic matching layer

11. A probe which is an array element probe used for ultrasound diagnosis equipment, wherein a piezoelectric material molded body used for the probe is made of porous ceramic.

12. The probe including claim 11, wherein, in the porous ceramic, 1000 or more pores are present separately and independently from one another per cubic millimeter.

13. The probe including claim 11 or 12, wherein porosity of the porous ceramic is in a range of 0.1 to 15 %, preferably in the range of 0.1 to 10 %.

14. The probe including any one of claims 11 to 13, wherein material of the porous ceramic used is two-component PZT or three-component PZT.
